(19) Europäisches Patentamt
European Patent Office
Office européen des brevets

(11) Veröffentlichungsnummer: **0 150 740**
**B1**

(12) # EUROPÄISCHE PATENTSCHRIFT

(45) Veröffentlichungstag der Patentschrift: **16.09.87**

(51) Int. Cl.[4]: **C 07 C 11/02**, C 07 C 1/20

(21) Anmeldenummer: **85100255.0**

(22) Anmeldetag: **12.01.85**

(54) **Verfahren zur Herstellung von Olefinen aus Methanol.**

(30) Priorität: **21.01.84 DE 3402020**

(43) Veröffentlichungstag der Anmeldung: **07.08.85 Patentblatt 85/32**

(45) Bekanntmachung des Hinweises auf die Patenterteilung: **16.09.87 Patentblatt 87/38**

(84) Benannte Vertragsstaaten: **BE DE FR GB IT NL**

(56) Entgegenhaltungen:
**EP - A - 0 077 523**
**EP - A - 0 088 494**
**DE - A - 3 141 283**

(73) Patentinhaber: **HOECHST AKTIENGESELLSCHAFT, Postfach 80 03 20, D-6230 Frankfurt am Main 80 (DE)**

(72) Erfinder: **Ebertz, Wolfgang, Dr., Bismarckstrasse 25, D-5300 Bonn 1 (DE)**
Erfinder: **Leupold, Ernst Ingo, Dr., Am Zäunefeld 15, D-6392 Neu-Anspach (DE)**
Erfinder: **Schütz, Joachim, Dr., Kurhausstrasse 57a, D-6238 Hofheim am Taunus (DE)**
Erfinder: **Baltes, Herbert, Dr., Johannesallee 24, D-6230 Frankfurt am Main 80 (DE)**
Erfinder: **Litterer, Heinz, Dr., Hardtstrasse 77, D-6208 Bad Schwalbach (DE)**
Erfinder: **Wunder, Friedrich, Dr., Jahnstrasse 46, D-6093 Flörsheim am Main (DE)**

EP 0 150 740 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Olefinen aus Methanol an Zeolith-Katalysatoren in einem Hordenreaktor.

Aus DE-OS 2 755 299, US-PS 4 062 905, US-PS 3 911 041, US-PS 4 079 096, US-PS 3 979 472 sowie DE-OS 2 615 150 sind Verfahren bekannt, in denen niedere Alkohole an Zeolithen zu Olefinen umgesetzt werden. In EP-OS 060 103 ist die Verwendung polytrop betriebener Rohrbündelreaktoren bzw. isothermer Fliessbettreaktoren und die Aufarbeitung des Reaktionsgemisches aus der Methanolumsetzung an Zeolithen beschrieben, während die Verwendung eines Hordenreaktors als unvorteilhaft wegen der schwierigen Temperaturführung beschrieben wird. Ein Zweistufenprozess, bestehend aus einer Dehydratisierungsstufe von Methanol zu Dimethylether und Wasser und der anschliessenden Umsetzung des resultierenden etherreichen Gemisches an Zeolithen in einem adiabatisch betriebenen Hordenreaktor ist Gegenstand der EP-OS 088 494. Die Dehydratisierungsstufe vor der eigentlichen Umsetzung zu Olefinen wird zwingend vorgeschrieben. Ferner wird der Einsatz radial durchströmter Katalysatorschüttungen zur Verringerung des Druckabfalls und damit zur Selektivitätserhöhung beschrieben.

Es bestand daher die Aufgabe ein Verfahren zu entwickeln, das in einem Hordenreaktor in einer Stufe, d.h. ohne vorhergehende Dehydratisierungsstufe wirtschaftlich mit hoher Selektivität Methanol in niedere Olefine umwandelt.

Gegenstand der Erfindung ist daher ein Verfahren zur Herstellung von Olefinen aus Methanol an Zeolith-Katalysatoren, das dadurch gekennzeichnet ist, dass das Verfahren ausschliesslich in einem Hordenreaktor durchgeführt wird.

Im Hinblick auf den Stand der Technik gemäss der oben erwähnten EP-OS 0 088 494 und EP-OS 0 060 103 ist es überraschend, dass sich Methanol ohne vorhergehende separate Dehydratisierungsstufe bei adiabatischen Reaktionsbedingungen in einem Hordenreaktor mit hoher Selektivität zu niederen Olefinen umsetzen lässt; es war nicht vorhersehbar, dass sich die Temperaturführung in einem Hordenreaktor im Gegensatz zur Behauptung in der EP-OS 0 060 103 durchaus beherrschen lässt.

Das erfindungsgemässe Verfahren bietet den grossen wirtschaftlichen Vorteil der einstufigen Prozessführung und vermeidet zudem die bei zweistufigen Verfahren im Dehydratisierungsschritt stets in gewissem Umfang auftretende Spaltung von Methanol in CO und Wasserstoff. Gegenüber der polytropen Fahrweise im Rohrbündelreaktor bietet die Umsetzung im Hordenreaktor den Vorteil des geringeren Druckabfalls, was zur Erhöhung der Selektivität zu niederen Olefinen führt. Die hordenweise Anordnung des Zeolith-Katalysators ermöglicht, insbesondere bei Anwendung des Radialreaktorprinzips, einen leichten und sehr schnellen Wechsel des Katalysators und eine ungewöhnliche Flexibilität hinsichtlich wechselnder Katalysatorqualitäten oder -typen und der Anpassung an deren spezifische Umsatzcharakteristika.

Als Ausgangsmaterial des erfindungsgemässen Verfahrens wird dampfförmiges Wasser/Methanol-Gemisch eingesetzt, wobei der Wasseranteil 40 bis 80% m/m, vorzugsweise 50 bis 70% m/m beträgt. Da die Katalysatoraktivität im Laufe der Reaktionsdauer nachlässt, enthält der Reaktoraustrag wechselnde Mengen Dimethylether. Dieser Ether kann nach Abtrennung dem Einsatzgemisch hinzugefügt werden und 0 bis 25% m/m, vorzugsweise 0 bis 15% m/m der Gesamteinsatzmischung betragen.

Die LHSV des Gesamteinsatzgemisches wird im allgemeinen zwischen 0,5 und 20 $h^{-1}$, vorzugsweise 2 bis 15 $h^{-1}$ gewählt.

Als Zeolith-Katalysatoren kommen vor allem solche vom Pentasiltyp in Betracht. Für den Begriff Pentasile gilt dabei die Definition von Kokotailo und Meier («Pentasil family of high silicon crystalline materials» in Special Publication n. 33 of the Chemical Society London 1980): Die Pentasil-Familie umfasst beispielsweise die synthetischen Zeolithe ZSM-5 (US-PS 3 702 886), ZSM-8 (GB-PS 1 334 243, ZMS-11 (US-PS 3 709 979) und ZMS-23 (US-PS 4 076 842).

Bevorzugt werden titan-, zirkon- und/oder hafniumhaltige Pentasile verwandt, wie sie in DE-OS 3 141 283 und DE-OS 3 141 285 beschrieben sind. Von diesen sind geeignet vor allem Zirkono-, Titano- und/oder Hafno-Silicate bzw. -Aluminosilicate mit ZSM-5-Struktur, vorzugsweise solche mit folgender Zusammensetzung, ausgedrückt in Molverhältnissen der Oxide:

$SiO_2 : (0 - 0,15)\ Al_2O_3 : (0,002 - 1,0)\ MO_2$,

insbesondere

$SiO_2 : (0,005 - 0,1)\ Al_2O_3 : (0,01 - 0,4)\ MO_2$,

wobei M gleich Zirkon, Titan und/oder Hafnium ist.

Diese Zeolithe lassen sich nach Methoden herstellen, wie sie auch für die Synthese des zirkon-, titan- bzw. hafniumfreien Zeolithen ZSM-5 beschrieben wurden, beispielsweise unter Verwendung von Alkylammoniumsalzen (US-PS 3 702 886), von Trialkylaminen bei gleichzeitiger Anwendung von Alkylierungsmitteln (DE-AS 2 212 810), von Diaminen (DE-OS 2 831 334) und/oder von Impfkristallen in Gegenwart oder Abwesenheit von Alkoholen und/oder Ammoniumhydroxid (US-PS 4 199 556).

Ein bevorzugtes Verfahren zur Synthese der genannten Zeolithe besteht darin, dass man Zirkon-, Titan- und/oder Hafnium- sowie Silizium-, Natrium- und Tetrapropylammoniumverbindungen, sowie im Falle der Aluminiumsilicate noch zusätzlich Aluminiumverbindungen, mit Wasser mischt und dieses Gemisch in einem geschlossenen Gefäss erhitzt. Diesem Gemisch können darüberhinaus vor dem Erhitzen Impfkristalle zugesetzt werden.

Die Ausgangsverbindungen werden im allgemeinen in folgendem Verhältnis eingesetzt, ausgedrückt in Molverhältnissen der Oxide:

$SiO_2 : (0\text{-}0,2)\ Al_2O_3 : (0,01\text{-}1,0)\ MO_2$:
$(0,01\text{-}0,5)\ Na_2O : (0,02\text{-}1,0)\ R_2O : (5\text{-}100)\ H_2O$,

vorzugsweise im Verhältnis

$SiO_2 : (0,01\text{-}0,1)\ Al_2O_3 : (0,01\text{-}0,4)\ MO_2$:
$(0,02\text{-}0,3)\ Na_2O : (0,03\text{-}0,6)\ R_2O : (10\text{-}40)\ H_2O$,

wobei M gleich Zirkon, Titan und/oder Hafnium und R gleich Tetrapropylammonium ist.

Als Verbindungen können beispielsweise eingesetzt werden: Kieselsäuregel, Natriumsilicat, Aluminiumhydroxid, Aluminiumsulfat, Natriumaluminat, Aluminiumhalogenide, Aluminiummetahydroxid, Zirkonhalogenide, Zirkonsulfat, Zirkonylchlorid, Titanhalogenide, Titansulfat, Hafniumhalogenide, Hafniumsulfat, Natriumhydroxid, Natriumsulfat, Natriumhalogenide, Tetrapropylammoniumhydroxid, Tetrapropylammoniumhalogenide. Aber auch andere Silizium-, Aluminium-, Zirkon-, Titan-, Hafnium-, Natrium- und Alkylammoniumverbindungen eignen sich für die Herstellung der Zeolithe.

Das Gemisch der jeweils gewählten Verbindungen mit Wasser wird im allgemeinen 18 bis 360 Stunden, vorzugsweise 24 bis 240 Stunden lang auf eine Temperatur zwischen 100 und 200°C, vorzugsweise zwischen 130 und 170°C, in einem geschlossenen Gefäss erhitzt.

Die gebildeten Zeolithe werden in üblicher Weise, z.B. durch Filtration, isoliert, gewaschen und getrocknet. Sie können nach bekannten Methoden in die katalytisch aktiven Formen überführt werden, z.B. durch Kalzinierung und/oder Ionenaustausch (D.W. Breck, Zeolithe Molecular Sieves, 1974).

Es war nicht vorhersehbar, dass insbesondere die Ti-, Zr- und/oder Hf-haltigen Pentasile bei dem erfindungsgemässen einstufigen Verfahren eine besonders hohe Selektivität zu $C_2$ bis $C_4$-Olefinen aufweisen. Die Eintrittstemperatur beträgt bei jeder der Horden 270 bis 400°C. Die Eintrittstemperaturen in den einzelnen Horden können unabhängig voneinander gewählt werden. Sie können während der Reaktion konstant gehalten oder bei Nachlassen der Katalysatoraktivität innerhalb des genannten Temperaturbereiches gesteigert werden. Zur Einhaltung der gewählten Temperaturen wird zwischen den Horden mittels indirekter oder direkter (Einspritz-) Kühlung die Reaktionswärme abgeführt. Bevorzugt wird durch Einspritzen von flüssigen Methanol/Wasser-Gemischen mit einem Wasseranteil von 40 bis 80% m/m zwischen den Horden die Reaktionswärme zur Bildung von Kohlenwasserstoffen genutzt. Der Druck ist so zu wählen, dass am Reaktorausgang noch 1,5 bis 10 bar, vorzugsweise 1,5 bis 4,5 bar Druck herrschen.

Die Katalysatoranordnung erfolgt im allgemeinen in 3 bis 10, bevorzugt in 4 bis 8 Horden. Die Schüttungen können in klassischer Weise axial durchströmt werden, aus konstruktiven und wirtschaftlichen Gründen wird jedoch dem Prinzip des Radialhordenreaktors der Vorzug gegeben. Die Schüttungshöhen in Strömungsrichtung ergeben sich aus den Umsatzcharakteristika der eingesetzten Zeolithe und betragen im allgemeinen 0,1 bis 2 m, bevorzugt 0,2 bis 1,6 m.

Das am Ausgang der letzten Horde austretende Reaktionsgemisch wird in Gas- und Flüssiganteil getrennt und der Gasanteil, bestehend aus $C_1$- bis $C_4$-Kohlenwasserstoffen und Dimethylether nach gängigen Verfahren der Absorption oder Rektifikation von Dimethylether befreit, der im alllgemeinen zurückgeführt wird. Die Aufarbeitung des verbleibenden Kohlenwasserstoffgemisches geschieht nach bewährten Methoden der Raffinerietechnik. Aus der Flüssigphase wird der wässrige Anteil isoliert und das darin enthaltene nicht umgesetzte Methanol und geringe Mengen Dimethylether zum Reaktor zurückgeführt. Die in geringer Menge anfallenden $C_5^+$-Kohlenwasserstoffe können entweder zu Vergaserkraftstoffen oder zu Chemierohstoffen aufgearbeitet werden.

Der hohe Anteil an $C_8$-Aromaten im organischen Teil der Flüssigphase und hierin der aussergewöhnlich hohe p-Xylolanteil geben der Aufarbeitung zu Chemierohstoffen eine besondere wirtschaftliche Bedeutung.

Die Durchführung des erfindungsgemässen Verfahrens in einer besonders günstigen Ausführungsform, nämlich mit parallel zueinander angeordneten Horden, wird durch Figur 1 erläutert.

Aus dem Vorratsgefäss 1 wird Methanol über Leitung 2 in den Mischer 3 geleitet und dort mit zurückgeführtem wässrigem Methanol aus Leitung 4 gemischt. Diese Mischung wird über Leitung 5 in die Verdampfer-Mischer-Einheit 6 geleitet, wo sie nach Verdampfung mit zurückgeführtem Dimethylether aus Leitung 7 gemischt wird. Die Mischung aus Wasser, Methanol und Dimethylether gelangt über Leitung 8 in den Wärmetauscher 9, wird dort auf 270 bis 400°C erwärmt und über Leitung 10 der Horde 11 a zugeführt. Aus Leitung 5 wird über die Leitungen 12-15 flüssiges Wasser-Methanol-Gemisch in die Leitungen 16-19 zwischen den Horden 11 a-e eingespritzt. Das Reaktionsprodukt, bestehend aus Wasser, olefinreichem Kohlenwasserstoffgemisch, Dimethylether und nicht-umgesetztem Methanol wird über Leitung 20 in den Wärmetauscher 21 geleitet und dort auf eine Temperatur abgekühlt, die es erlaubt, das Reaktionsprodukt nach Durchlaufen von Leitung 22 im Wärmetauscher 9 zur Erhitzung der Einsatzmischung auf die Reaktoreintrittstemperatur zu benutzen. Das Reaktionsprodukt verlässt durch Leitung 23 den Wärmetauscher 9 und gelangt in den Separator 24. Dort werden die Gasphase 25, die $C_5^+$-Kohlenwasserstoffphase 26, und die methanolhaltige Wasserphase 27 getrennt. Die Wasserphase enthält im allgemeinen 0 bis 20% m/m Methanol, geringe Mengen Dimethylether, sowie Spuren von Essigsäure, Methylacetat und Aceton, die den Prozess jedoch nicht negativ beeinflussen. Ein Teil der Wassephase 27 wird zur Herstellung der Einsatzmischung über Leitung 4 dem Mischer 3 zugeführt. Der Rest der Wasserphase, nämlich soviel, wie der durch Kohlenwasserstoffbildung entstandenen Wassermenge entspricht, wird durch Leitung 28 in Kolonne 29 geleitet, wo das Methanol vom Wasser abdestilliert wird. Das Methanol wird durch Leitung 30 in das Vorratsgefäss 1 zurückgeführt. Das Wasser wird über Leitung 31 entnommen und verworfen. Die $C_5^+$-Phase 26 wird über Leitung 32 entnommen und einer Aufarbeitung zu Vergaserkraftstoff oder Chemierohprodukten zugeführt. Die Gasphase 25, bestehend aus $C_1$- bis $C_4$-Kohlenwasserstoffen (hauptsächlich ungesättigten), Dimethylether sowie geringen Mengen CO, $H_2$ und $CO_2$ wird durch Leitung 33 in Trennstufe 34 geleitet und dort durch Destillation oder Absorption-Desorption vom Dimethylether befreit, der durch Leitung 7 zurückgeführt

wird. Das vom Ether befreite Produkt wird durch Leitung 35 entnommen und nach bekannten Verfahren zu reinen $C_2$- bis $C_4$-Olefinen aufgearbeitet.

Die Exothermie der Reaktion begrenzt stark die realisierbaren Katalysator-Schütthöhen. Daher wird es notwendig, den Katalysator in geringer Schütthöhe grossflächig auszubreiten. Die beim Radialreaktor zylindrische Anordnung des Katalysators erlaubt im Vergleich zur planen Anordnung eine Unterbringung grosser Anströmflächen auf geringem Raum. Ausserdem entfallen die bei planer Anordnung erforderlichen aufwendigen Massnahmen zur gleichmässigen Verteilung des Produktstroms über die gesamte Schüttung. Zudem ergibt sich bei radialer Bauweise der Horden der Vorteil, dass man diese parallel anordnen kann (Fig. 1). Die hierdurch erreichte geringe Bauhöhe des Gesamtreaktors und die gute Zugänglichkeit der einzelnen Hordenfüllungen ermöglicht einen schnellen Katalysatorwechsel. Insbesondere kann man die Füllungen ausserhalb der Horden mit gleichmässiger Schüttdichte in allen Teilen der Füllung herstellen.

Fig. 2 zeigt den Längsschnitt der Horde 11 a eines in radialer Bauweise zylindrisch ausgeführten Hordenreaktors 11 a-e. Durch Leitung 10 gelangt die Einsatzmischung aus Wasser, Methanol und Dimethylether in den Eintrittskanal 36 der Horde 11 a. In radialer Richtung tritt der Reaktandenstrom durch die Katalysator-Füllung 38, die von den perforierten konzentrischen Anströmflächen 37 und 39 begrenzt ist. Dann wird der Gasstrom in den Austrittskanal 40 umgelenkt und tritt durch Leitung 16 mit erhöhter Temperatur (wegen der Exothermie) aus der Horde 11 a aus. Zur Erniedrigung der Eintrittstemperatur der folgenden Horde wird durch Leitung 12 flüssiges Methanol-Wasser-Gemisch zugeführt und im statischen Mischer 41 eine homogene Verteilung der Komponenten und der Temperatur erreicht, bevor die Reaktionsmischung über Leitung 16 der folgenden Horde zugeführt wird.

Eine wirtschaftlich besonders günstige Dimensionierung einer solchen Radialhorde 11 a wird erreicht, wenn die Querschnittsfläche Fe des Eintrittskanals 36 und die Querschnittsfläche Fa des Austrittskanals 40 klein sind gegenüber der gesamten mittleren Anströmfläche Fk der zylindrischen Katalysator-Füllung 38. Diese Flächen ergeben sich aus den Formeln

$$Fe = \frac{\Pi E^2}{4},\ Fa = \frac{\Pi}{4}[A^2-(E+2K)^2],\ Fk = \Pi(E+K)H$$

wobei Fk sich auf die geometrische Mitte der zylindrischen Katalysator-Füllung 38 bezieht. Dabei bedeutet H die Höhe der Füllung 38 und K deren Dicke, E den Durchmesser des Eintrittskanals 36 und A den Durchmesser des Austrittskanals 40.

Vorzugsweise ist $\frac{Fe}{Fk}$ = 0,02 bis 0,1 und $\frac{Fa}{Fk}$ = 0,04 bis 0,2, insbesondere $\frac{Fe}{Fk}$ = 0,05 bis 0,08 und $\frac{Fa}{Fk}$ = 0,1 bis 0,15. Wenn man innerhalb dieser Bereiche arbeitet, erzielt man eine sehr gleichmässige Durchströmungsgeschwindigkeit.

Das erfindungsgemässe Verfahren bietet die Möglichkeit, unabhängig von der bisher betriebenen Spaltung leichter Naphtha-Schnitte aus der Rohölraffination, ausgehend von Erdgas, Kohle u.ä. vergasungsfähigen Rohstoffen, mit höherer Selektivität als bei der Naphthaspaltung zu niederen Olefinen zu gelangen.

Die folgenden Beispiele sollen die Erfindung erläutern, aber in keiner Weise einschränken.

A) *Allgemeine Versuchsbedingungen*

Die Apparatur besteht aus einem axial durchströmten Hordenreaktor mit 4 rohrförmigen Horden (100 mm innerer Durchmesser) aus korrosionsfestem Stahl, in dem mit jeweils mehreren Thermoelementen die Katalysatortemperatur in direktem Kontakt gemessen und registriert wird. Der Reaktor wird von der Aussenheizung auf Reaktionstemperatur gebracht und ein allseitig abgedichteter Spaltraum gewährleistet eine adiabatische Betriebsweise.

Die Einsatzmischung wird durch einen Verdampfer und einen Überhitzer auf die erste Schüttung geführt. Zur Abfuhr der Reaktionswärme wird zwischen den Horden Methanol/Wasser-Gemisch in solcher Menge geregelt eingespeist, dass nach Durchlaufen einer Mischstrecke die vorgeschriebene Eintrittstemperatur auf der nächsten Schüttung erreicht wird. An den Augang der letzten Horde schliesst sich ein Wärmetauscher an, der das Reaktionsgemisch kühlt und in Gas- und Flüssigphase trennt. Die Gasphase wird volumetrisch erfasst und onlinegaschromatographisch analysiert. Die Flüssigphase wird gewogen, die $C_5^+$-Kohlenwasserstoffphase abgetrennt und gewogen. Beide Flüssigphasen werden offline analysiert.

Der Reaktor bietet die Möglichkeit, nach den einzelnen Horden die Gas- und Flüssigkeitszusammensetzungen zu untersuchen.

B) *Versuchsergebnisse*

Vergleichsbeispiel und Beispiel 1:

Es wird Methanol/Wasser im Verhältnis 1:2 m/m eingesetzt. Die Zusammensetzung der Reaktorausträge ist in Tabelle 1 dargestellt.

Im Vergleichsbeispiel wird ein gemäss Beispiel 1 der US-PS 3 702 886 hergestellter H-ZSM 5-Zeolith verwendet.

Im Beispiel 1 wird ein gemäss Beispiel 1 der DE-OS 3 141 285 wie folgt hergestellter Zirkonpentasil eingesetzt.

1,66 g Natriumaluminat (54% m/m $Al_2O_3$, 41% m/m $Na_2O$) und 1,48 g Natriumhydroxid werden in 20 g 20% m/m wässriger Tetrapropylammoniumhydroxid-Lösung gelöst (Lösung A). Eine weitere Lösung (Lösung B) wird hergestellt, indem man 62 g 40% m/m kolloidales Kieselgel in 230 g 20% m/m wässriger Tetrapropylammoniumhydroxid-Lösung löst und diese Lösung am Rotationsverdampfer auf insgesamt 220 g einengt. Lösung A und Lösung B werden miteinander vermischt. Zu dieser Mischung

werden unter intensivem Rühren 3,78 g Zirkonylchlorid $ZrOCl_2 \cdot 8\ H_2O$ gegeben. Die entstandene Suspension wird homogenisiert und in einem geschlossenen Gefäss 120 h auf 160°C erhitzt. Das entstandene Produkt wird abfiltriert, mit Wasser gewaschen und bei 120°C getrocknet. Man erhält 27,3 g Zirkonoaluminosilicat.

Die Röntgenbeugungsanalyse zeigt ein gut kristallines Produkt mit ZSM-5-Struktur. Die chemische Analyse des 16 Stunden bei 540°C kalzinierten Produktes zeigt folgende Zusammensetzung, ausgedrückt in Molverhältnissen der Oxide:

$SiO_2 : 0,035\ ZrO_2 : 0,026\ Al_2O_3 : 0,023\ Na_2O$.

Die Reaktionstemperatur beträgt im Vergleichsbeispiel und in Beispiel 1 jeweils 330°C an den Hordeneingängen, und in beiden Fällen beträgt die Gesamt-LHSV $5 \cdot h^{-1}$.

TABELLE 1

Zusammensetzungen der Reaktorausträge (% m/m) [1)]

| | Vergleichsbeispiel | Beispiel 1 |
|---|---|---|
| Dimethylether | 36,5 | 29,8 |
| CO, $CO_2$, $H_2$ | 0,4 | 0,9 |
| Methan | 0,6 | 1,1 |
| Ethan | 0,1 | 0,1 |
| Propan | 3,7 | 2,9 |
| Butane | 6,9 | 7,8 |
| $C_5^+$ | 12,7 | 10,6 |
| Aromaten | 8,5 | 8,0 |
| Ethylen | 21,7 | 25,6 |
| Propylen | 13,3 | 18,2 |
| Butene | 4,6 | 2,9 |
| Methanol-Umsatz zu Kohlenwasserstoffen (%) | 51 | 50 |

[1)] Die angegebenen Werte entsprechen einem 12 Stunden-Durchschnitt.

**Patentansprüche**

1. Verfahren zur Herstellung von Olefinen aus Methanol an Zeolith-Katalysatoren, dadurch gekennzeichnet, dass das Verfahren ausschliesslich in einem Hordenreaktor durchgeführt wird.

2. Verfahren nach Anspruch 1, dadurch gekennzeichnet, dass titan-, zirkon- und/oder hafniumhaltige Zeolithe vom Pentasil-Typ als Katalysatoren verwendet werden.

3. Verfahren nach Anspruch 1 oder 2, dadurch gekennzeichnet, dass die Zahl der Horden im Reaktor 3 bis 10 beträgt.

4. Verfahren nach einem der Ansprüche 1 bis 3, dadurch gekennzeichnet, dass der Produktstrom nach Kühlung hinter den Hordenaustritten intensiv gemischt wird.

5. Verfahren nach einem der Ansprüche 1 bis 4, dadurch gekennzeichnet, dass der Katalysator in radialer Richtung durchströmt wird.

6. Verfahren nach Anspruch 5, dadurch gekennzeichnet, dass die Horden mit radial durchströmten Katalysatorbetten parallel zueinander angeordnet sind.

**Claims**

1. A process for the production of olefins from methanol at zeolite catalysts, characterized by carrying out the process exclusively in a tray-type reactor.

2. The process as claimed in claim 1, wherein pentasil-type zeolites containing titanium, zirconium and/or hafnium are used as the catalysts.

3. The process as claimed in claim 1 or 2, wherein the number of trays in the reactor is 3 to 10.

4. The process as claimed in any of claims 1 to 3, wherein the product stream is intensively mixed after cooling downstream of the tray outlets.

5. The process as claimed in any of claims 1 to 4, wherein the flow through the catalyst is in the radial direction.

6. The process as claimed in claim 5, wherein the trays are arranged mutually parallel, with radial flow in the catalyst beds.

**Revendications**

1. Procédé pour préparer des oléfines à partir du méthanol sur des catalyseurs zéolitiques, procédé caractérisé en ce qu'on conduit le procédé en opérant directement dans un réacteur à plateaux.

2. Procédé selon la revendication 1, caractérisé en ce qu'on utilise comme catalyseurs des zéolites du type pentasil contenant du titane, du zirconium et/ou de l'hafnium.

3. Procédé selon la revendication 1 ou 2, caractérisé en ce que le nombre des plateaux est de 3 à 10 dans le réacteur.

4. Procédé selon l'une des revendications 1 à 3, caractérisé en ce qu'on mélange intensément le courant des produits, après son refroidissement en aval des sorties des plateaux.

5. Procédé selon l'une des revendications 1 à 4, caractérisé en ce que le catalyseur est traversé et parcouru en direction radiale.

6. Procédé selon la revendication 5, caractérisé en ce que les plateaux sont disposés parallèlement les uns aux autres en comportant des lits de catalyseurs traversés par des courants radiaux.

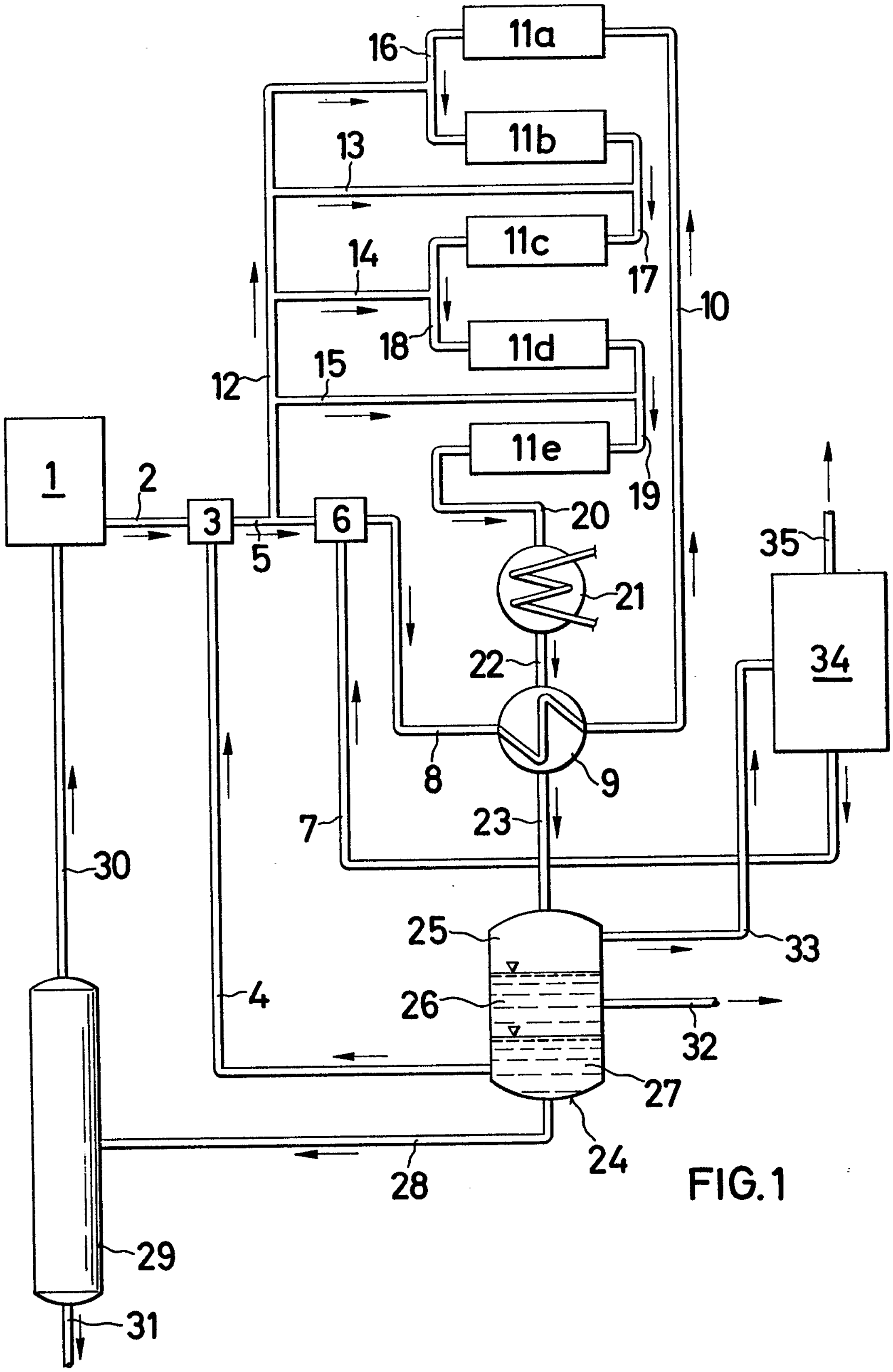
11a
11b
11c
11d
11e
1
2
3
4
5
6
7
8
9
10
12
13
14
15
16
17
18
19
20
21
22
23
24
25
26
27
28
29
30
31
32
33
34
35

FIG.1

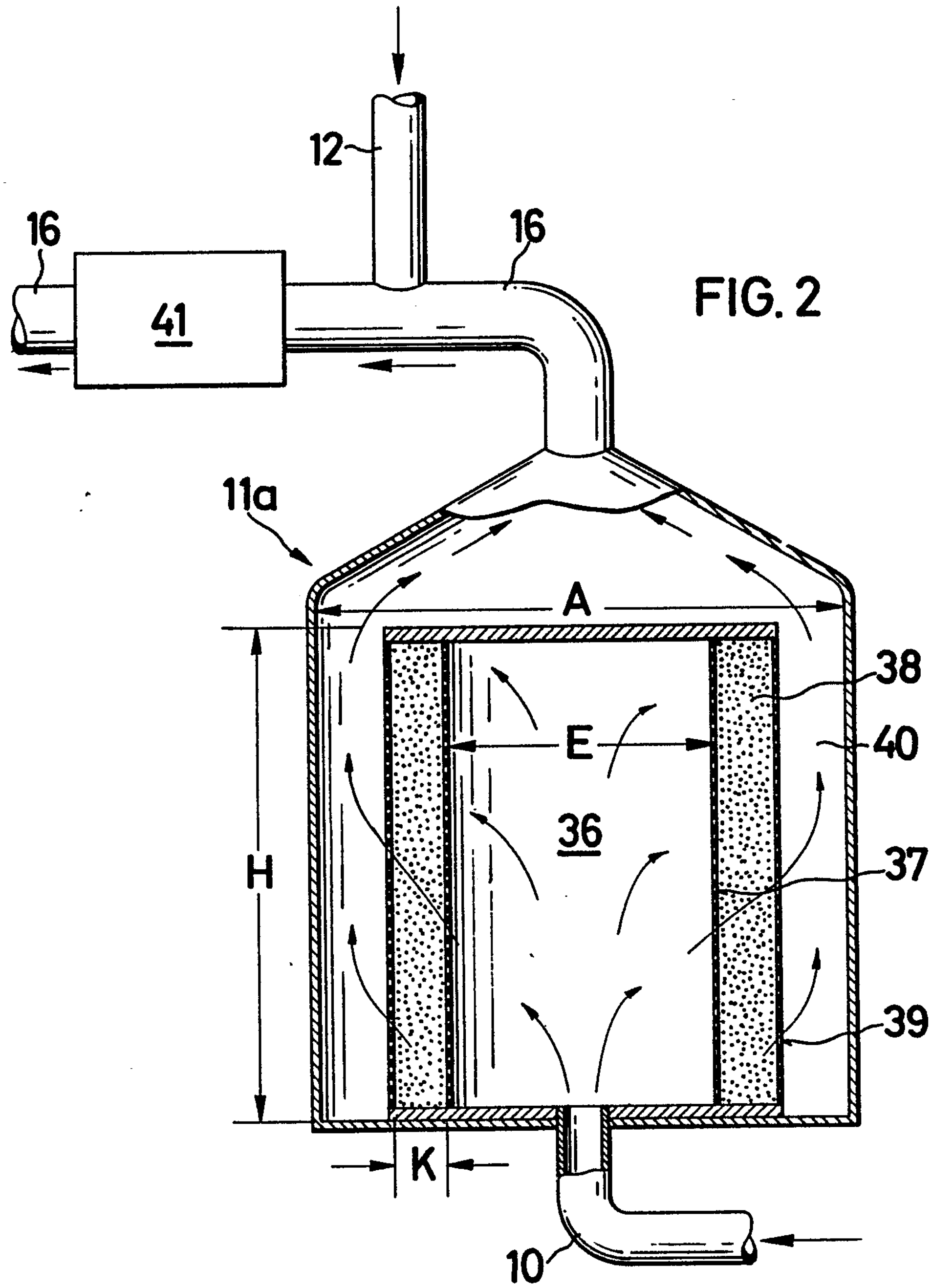


FIG. 2